Europäisches Patentamt
European Patent Office
Office européen des brevets

(11) Publication number: 0 280 461 A2

# EUROPEAN PATENT APPLICATION

(21) Application number: 88301286.6

(22) Date of filing: 17.02.88

(51) Int. Cl.4: C07C 91/32, C07C 89/04, A61K 31/135

The title of the invention has been amended (Guidelines for Examination in the EPO, A-III, 7.3).

(30) Priority: 24.02.87 US 18264

(43) Date of publication of application: 31.08.88 Bulletin 88/35

(84) Designated Contracting States: AT BE CH DE ES FR GB GR IT LI LU NL SE

(71) Applicant: ELI LILLY AND COMPANY
Lilly Corporate Center
Indianapolis Indiana 46285(US)

(72) Inventor: Hayes, James Scott
3508 Busseron Lane
Indianapolis Indiana 46220(US)
Inventor: Rolski, Stanislaw
8326 East Goldfinch Circle
Indianapolis Indiana 46256(US)

(74) Representative: Tapping, Kenneth George et al
Erl Wood Manor
Windlesham Surrey, GU20 6PH(GB)

## (54) Dobutamine salts.

(57) Dobutamine phosphate monobasic has desirable aqueous solubility properties, and is therefore useful in preparing intravenous solutions for treatment of cardiac insufficiency.

EP 0 280 461 A2

**IMPROVEMENTS IN OR RELATING TO DOBUTAMINE SALTS**

This invention relates to a dobutamine salt which has valuable solubility properties, and processes for making such salt.

Dobutamine is the generic name for (±)-N-[3-(4-hydroxyphenyl)-1-methylpropyl]-2-(3,4-dihydroxyphenyl)-ethylamine. Tuttle et al., U.S. Patent 3,987,200, disclose administering aqueous dobutamine hydrochloride or hydrobromide solutions to warm-blooded animals to increase cardiac contractility.

Unfortunately, the hydrochloride and hydrobromide salts of dobutamine exhibit relatively low aqueous solubility; namely, about 28 mg per ml of water at room temperature (25°C). This low solubility limits the use of these salts in liquid formulations, especially in applications requiring solutions containing high concentrations of dobutamine.

According to the present invention there is provided dobutamine phosphate monobasic, which exhibits greatly increased aqueous solubility properties compared to previously disclosed dobutamine salts. Thus, pharmaceutical formulations containing such salt exhibit improved stability characteristics compared to the previously known salts of dobutamine.

Dobutamine is a racemate of the following structure:

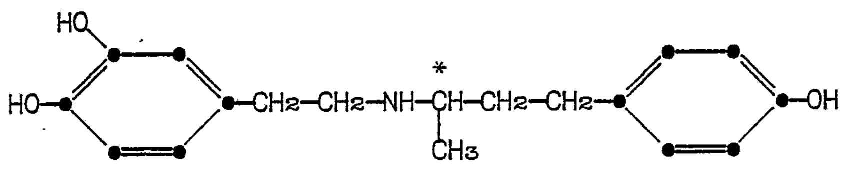

The α-carbon (marked with an asterisk) is asymmetric, yielding two stereoisomers, the (+) and (-), which, in a 1:1 mixture, form the racemate. This invention includes soluble phosphate monobasic salts of both the (+) and (-) stereoisomer, as well as the racemate. The term "dobutamine" as used herein includs both the racemate and individual stereoisomers.

The dobutamine phosphate salt of this invention can be prepared by direct neutralization of the amine base or by a metathetic reaction (double decomposition) involving an acid addition salt of dobutamine. The neutralization procedure is preferred.

According to one aspect of the invention there is provided a process for preparing a soluble salt of dobutamine; namely, dobutamine phosphate monobasic, which comprises:

a) neutralizing dobutamine as the free base with phosphoric acid; or

b) the metathetic reaction of an acid addition salt of dobutamine with phosphoric acid.

In the neutralization procedure, the dobutamine phosphate monobasic salt of this invention can be prepared by reacting dobutamine, as the free amine, with phosphoric acid. The free amine and phosphoric acid are employed in equimolar quantities to provide a solution whose pH in water is about 3.5 to about 4.0. The reaction can be conducted in water, or inert organic solvents, for example, alcohols such as methanol, ethanol, isopropanol, or the like. The solid salt can be obtained from the solution by either freeze drying, evaporating the solvent or filtration. Isopropanol is the preferred solvent for preparing the salt of the invention since dobutamine phosphate monobasic precipitates during neutralization of dobutamine base with phosphoric acid and is easily recovered by standard filtration techniques.

Dobutamine can form two salts upon reaction with phosphoric acid; namely, dobutamine phosphate monobasic and dobutamine phosphate dibasic. Dobutamine phosphate monobasic is formed when one mole of phosphoric acid reacts with one mole of dobutamine. The dibasic salt forms when one mole of phosphoric acid reacts with two moles of dobutamine.

Dobutamine phosphate dibasic exhibits greatly decreased aqueous solubility compared to the monobasic salt form, about 25 mg per ml of water at 25°C. To avoid forming dobutamine phosphate dibasic when preparing the monobasic salt of the invention the pH of the reaction solution should be lowered until it is about 3.5 to about 4.0. The solution's pH can be followed during neutralization by diluting an aliquot of the reaction solution with a 5-fold portion of water and measuring pH.

Alternatively, the dobutamine phosphate monobasic salt of this invention can be prepared by metathetic reaction. In the metathetic reaction procedure, an acid addition salt of dobutamine, such as dobutamine hydrochloride, can be dissolved in an excess of phosphoric acid, for example 5N $H_3PO_4$. The resulting mixture is concentrated to dryness by evaporation. The dobutamine phosphate salt can then be obtained in solid form by precipitating it from an inert organic solvent. for example, isopropanol.

Starting materials required for preparing the salt of the invention are known. Preparation of the free amine can be done according to conventional procedures as described in European Patent Application 187,019. Preparation of acid addition salts of dobutamine can also be done according to conventional procedures as described in United States Patent No. 3,987,200 and the above mentioned European Patent Application.

The following Examples illustrate specific aspects of the present invention. The Examples are not intended to limit the scope of the invention in any respect and should not be so construed.

Example 1

Preparation of dobutamine free base

Dobutamine hydrochloride (2.0 g) and sodium bisulfate (30.0 mg) were dissolved in 150 ml of deaerated water. To this solution was added dropwise, with stirring, a 10% aqueous ammonia solution until the pH reached 9.0. Dobutamine free base precipitated and was collected by filtration through a sintered glass filter. The solid was washed with a small amount of deaerated, deionized water and dried under vacuum at room temperature (25°C) to yield 1.48 g of dobutamine free base. Both the reaction and filtration were carried out under nitrogen to maintain an oxygen free atmosphere.

Example 2

Preparation of dobutamine phosphate monobasic

Dobutamine free base (1.0 g), prepared according to Example 1, was dissolved in 150 ml of isopropanol. A 5% solution of crystalline phosphoric acid dissolved in isopropanol was added dropwise, with stirring, until the pH of the solution reached 4.0 (pH was measured on an aliquot diluted with a 5-fold portion of water). The precipitate obtained was filtered through a sintered glass filter and dried under vacuum at 45°C to provide 973 mg of dobutamine phosphate.

Analysis calc. for $C_{18}H_{26}NO_7P$

Theory:

C, 54.13; H, 6.56: N, 3.52; P, 7.76

Found:

C, 54.36; H, 6.50; N, 3.24; P, 7.50

Example 3

Preparation of dobutamine phosphate monobasic

Dobutamine free base (1.5 g) was dissolved in 200 ml of isopropanol. A 5% solution of crystalline phosphoric acid dissolved in isopropanol was added dropwise, with stirring, until the pH reached 3.5 (pH was measured on an aliquot diluted with a 5-fold portion of water). The precipitate that formed was filtered through a sintered glass filter and dried under vacuum at room temperature to provide 1.345 g of dobutamine phosphate.

Analysis calc. for $C_{18}H_{26}NO_7P$

Theory:

C, 54.13; H, 6.56; N, 3.51; P, 7.76

Found:

C, 54.31; H, 6.39; N, 3.35; P, 7.48

The aqueous solubility of the dobutamine phosphate salt of the invention is about 2.0 g per ml of water, at 25°C. In contrast, a saturated solution of dobutamine hydrochloride provides only 28 mg per ml of water at 25°C. The increased solubility of the invention salt allows the preparation of pharmaceutical formulations which have improved stability characteristics. The increased solubility also allows formulation of solutions containing greatly increased concentrations of dobutamine. Increased solubility is important in applications requiring small liquid volumes, such as implantable mini-pumps capable of continuously administering dobutamine for long durations.

The dobutamine phosphate monobasic salt of this invention is useful for increasing cardiac contractility in warm-blooded animals suffering from acutely depressed cardiac contractility. Dobutamine phosphate can be administered by intravenous infusion at a rate of about 0.5 to about 10.0 mcg/kg/min until contractile force of the heart muscle is restored. The rate of infusion may either be decreased or discontinued as normal contractility is restored. In certain instances a particular host may require continuous administration for long durations; for example a few days, whereas with other hosts a single infusion of a short duration may suffice to produce the desired restoration of normal contractility.

Dobutamine phosphate monobasic salt, when administered according to the above method, exerts a positive inotropic effect on the heart without inducing arrhythmia or increasing heart rate. In addition, compounds of the present invention do not restrict blood flow to vital organs, nor do they have an adverse affect on the central nervous system.

The (-)-dobutamine phosphate monobasic salt, when adminiistered according to the above method, is especially useful in the treatment of acutely depressed cardiac contractility complicated by marked hypotension, since it possesses more pressor activity than either the racemate or the (+)-stereoisomer.

The phosphate salt of the invention can be formulate with any number of pharmaceutical carriers according to recognized techniques. A typical injectable (i.v.) formulation using dobutamine phosphate has the following composition:

500 mg (base equivalent) dobutamine phosphate
5 mg sodium bisulfite
water (WFI, deoxygenated) q.s. to 5 ml.

Such solutions can be sterilized by standard methods, such as sterile filtration, and placed in 5 ml glass ampoules, or glass vials with rubber stoppers, and the container sealed while maintaining an oxygen-free atmosphere. The ampoule can be opened as needed to provide an injectable solution for treatment of cardiogenic shock. Alternatively, the solutions can be lyophilized and reconstituted with WFI prior to use.

Other anti-oxidants may be used in place of sodium bisulfite. Examples of acceptable anti-oxidants include sodium sulfite, other alkali metal sulfites or bisulfites, thioglycerol, and thioerithritol. Other liquids such as glucose or saline solutions can be used in place of water.

Formulations containing dobutamine phosphate monobasic have superior stability characteristics. Accordingly, in one aspect of the invention there is provided a pharmaceutical formulation comprising a solution (preferably aqueous) of dobutamine phosphate monobasic and an anti-oxidant (preferably sodium bisulfite).

## Claims

1. A (1:1) salt of N-[3-(4-hydroxyphenyl)-1-methylpropyl]-2-(3,4-dihydroxyphenyl)ethylamine formed with phosphoric acid.

2. A salt as claimed in claim 1, said salt being racemic dobutamine phosphate monobasic.

3. A salt as claimed in claim 1, said salt being (-)-dobutamine phosphate monobasic.

4. A salt as claimed in claim 1, said salt being (+)-dobutamine phosphate monobasic.

5. A pharmaceutical formulation comprising a solution of a salt of dobutamine and phosphoric acid as claimed in any one of claims 1 to 4, associated with an anti-oxidant.

6. A pharmaceutical formulation as claimed in claim 5 wherein the solution is an aqueous solution.

7. A pharmaceutical formulation comprising an aqueous solution of racemic dobutamine phosphate monobasic and sodium bisulfite.

8. A pharmaceutical formulation comprising an aqueous solution of (-)-dobutamine phosphate monobasic and sodium bisulfite.

9. A salt of dobutamine and phosphoric acid as claimed in any one of claims 1 to 4 for use as a pharmaceutical compound.

10. A process for preparing a salt of dobutamine and phosphoric acid as claimed in any one of claims 1 to 4 which comprises:

a) neutralizing dobutamine as the free base with phosphoric acid; or

b) the metathetic reaction of an acid addition salt of dobutamine with phosphoric acid.

Claims for the following contracting States: GR, ES

1. A process for preparing a (1:1) salt of N-[3-(4-hydroxyphenyl)-1-methylpropyl]-2-(3,4-dihydroxyphenyl)ethylamine formed with phosphoric acid which comprises:

a) neutralizing dobutamine as the free base with phosphoric acid; or

b) the metathetic reaction of an acid addition salt of dobutamine with phosphoric acid.

2. A process as claimed in claim 1, wherein the salt prepared is racemic dobutamine phosphate monobasic.

3. A process as claimed in claim 1 wherein the salt prepared is (-)-dobutamine phsophate monobasic.

4. A process as claimed in claim 1 wherein the salt prepared is (+)-dobutamine phosphate monobasic.

5. A pharmaceutical formulation comprising a solution of a salt of dobutamine and phosphoric acid, as defined in any one of claims 1 to 4, associated with an anti-oxidant.

6. A pharmaceutical formulation as claimed in claim 5 wherein the solution is an aqueous solution.

7. A pharmaceutical formulation comprising an aqueous solution of salt of dobutamine and phosphoric acid, as defined in claim 2, and sodium bisulfite.

8. A pharmaceutical formulation comprising an aqueous solution of a salt of dobutamine and phosphoric acid as defined in claim 3, and sodium bisulfite.